# EUROPEAN PATENT SPECIFICATION

(11) **EP 4 236 865 B1**
(45) Date of publication and mention of the grant of the patent: **23.07.2025**
(21) Application number: 21787001.3
(22) Date of filing: 18.10.2021
(51) Int. Cl.: A61C 19/06, A61C 3/00, A61N 1/05, A61C 17/00, A61C 17/02, A61C 17/22

(54) **TOOTH REMINERALIZATION**
ZAHNREMINERALISIERUNG
REMINÉRALISATION DES DENTS

(30) Priority: 27.10.2020 EP 20204113
(43) Date of publication of application: 06.09.2023
(73) Proprietor: Koninklijke Philips N.V., 5656 AG Eindhoven (NL)
(72) Inventor: GOTTENBOS, Bart, 5656 AE Eindhoven (NL)
(74) Representative: Philips Intellectual Property & Standards
(86) International application number: PCT/EP2021/078737
(87) International publication number: WO 2022/089968

(56) References cited:
- WO-A2-2006/039278
- WO-A2-2014/009682
- US-A1- 2010 303 925

## Description

### FIELD OF THE INVENTION

The present invention relates to an oral treatment device for stimulating deposition of calcium phosphate from an amorphous calcium phosphate formulation in contact with a tooth in an oral cavity, the device comprising a first electrode, a second electrode and a power source.

The present invention further relates to a tooth remineralization kit comprising such a device and an amorphous calcium phosphate formulation.

The present invention yet further relates to an amorphous calcium phosphate formulation for use in a remineralizing treatment of a tooth.

### BACKGROUND OF THE INVENTION

US 2010/303925 A1 discloses cosmetic and therapeutic treatment of tissue, and in particular remineralizing tissue by applying a remineralizing agent whilst applying iontophoresis.

WO 2014/009682 A2 discloses an apparatus and method for mineralizing biological material by applying an ultrasonic signal and a mineralizing agent, and in some cases combining iontophoresis with ultrasound.

WO 2006/039278 A2 discloses a method and apparatus for tooth rejuvenation and hard tissue modification.

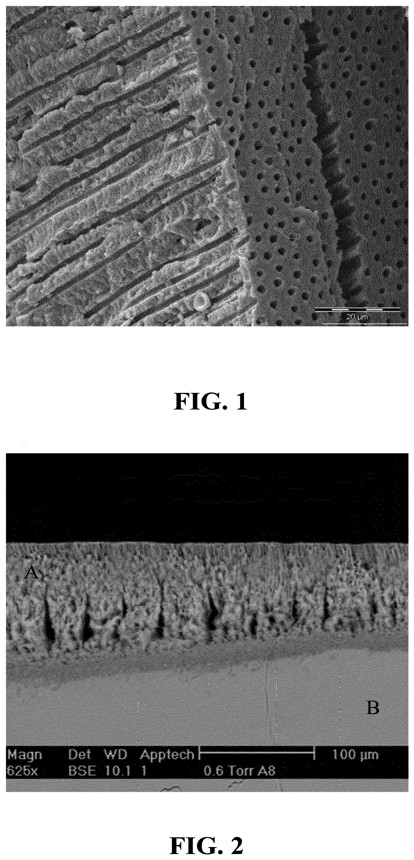

Amorphous calcium phosphate (ACP) formulations are widely used in the field of oral care to reduce tooth sensitivity, e.g. dentin hypersensitivity or sensitivity after tooth whitening. One mechanism by which these products reduce sensitivity is by depositing ACP particles on the teeth, which block porosities in the tooth enamel or dentin. Porosities are seen as one of the key causes of sensitive teeth, as they provide a hydrodynamic connection between the outside of the tooth and the tooth nerve, causing pain when putting pressure on the tooth, or by exposing the tooth to thermal stress, e.g. hot or cold temperatures causing expansion or contraction of the tooth. FIG. 1 is a scanning electron microscopy (SEM) of dentin showing the morphology of such porosities with pores having a length of around 4 mm extending from the outside towards the pulp containing the nerve, whereas FIG. 2 is a SEM image of a cross-section of carious enamel, with region A depicting the demineralized enamel and region B depicting the sound enamel. The demineralized enamel can have porosities of tens of microns length. The surface layer is typically denser, with porosities of a few microns in length. The sound enamel is very dense, but is still porous with submicron pores and the occasional wider cracks.

ACP can be further applied in the repair of demineralized tooth tissue, which causes the onset of caries. This also starts with depositing ACP particles on the affected tooth tissue, or ideally inside the demineralized porous enamel or dentin. The deposited calcium phosphate acts in multiple ways, remineralizing the tissue as it provides a reservoir for calcium and phosphate needed to rebuild the (partially) dissolved hydroxyapatite (HA) crystals of the carious tissue, and preventing new demineralization, as with an acid attack the ACP will first be sacrificed, protecting the natural HA from the tooth by providing high calcium and phosphate concentrations. Typically, fluoride is also added to such formulations, as it further protects against dissolution of hydroxyapatite and drives remineralization into fluoroapatate, which is more acid resistant.

Commercially available ACP relief gels are highly inefficient in leaving ACP attached to the tooth structures, and even more inefficient in getting it inside the porous target structures. The products work by combining two components, a calcium component and a phosphate component just before or while applying them on the teeth. Typically, such products are in some gel form, meaning the viscosity is high, which prevent them from hydrodynamically moving into porous tooth structures. Delivery of the calcium and phosphate into such porous structures has to rely on diffusion, a slow process, and therefore most of the ACP has already precipitated in the bulk of the gel, before even getting a chance to move inside a porous tooth structure. The deposition of ACP therefore only happens at the tooth interface with the gel, relying on attachment to the tooth surface. The bulk of the gel will be washed away, washing away the vast majority of ACP particles, only leaving the ones with sufficient attachment force to the tooth surface. Hence, this deposition process of ACP particles is rather inefficient.

Yu Yuan Zhang et al. in "Repair of dentine-related lesions without a drill or injection" in RSC Adv., 2019, 9, 15099, disclose a non-invasive system to improve the remineralization kinetics of a casein phosphopeptide (CPP)-ACP suspension by the application of an extrinsic electric field to acid-etched rabbit maxillary incisors using a personalized mold holding the suspension and a cathode, whilst the anode was attached to the skin on the rabbit's head. Upon application of a constant current of 0.5 mA or 1.0 mA, the acid-etched dentine of these incisors was fully remineralized after 5-8 hours depending on the applied current. This was attributed to the formation of hydroxide anions at the cathode through water hydrolysis, which locally increased the pH causing an increase of negative charge in CPP, with the extrinsic electric field causing migration of the CPP-ACP nano-complexes from the cathode into the acid-etched dentine through electrophoresis.

A drawback of the use of electrophoresis is that an extrinsic electric field needs to be deployed extending through the tooth, which can lead to discomfort in sensitive patients. Moreover, the use of an anode outside the oral cavity is cumbersome and potentially unpleasant for a patient, thus increasing the likelihood of poor compliance with a treatment regime.

### SUMMARY OF THE INVENTION

The present invention seeks to provide an oral treatment device that can promote ACP deposition at a tooth surface in a more user-friendly manner.

The present invention further seeks to provide a tooth remineralization kit comprising such an oral treatment device.

The present invention still further seeks to provide an amorphous calcium phosphate formulation for use in remineralizing a tooth.

The invention is defined by the independent claims. The dependent claims define advantageous embodiments.

According to an aspect, there is provided an oral treatment device for stimulating deposition of calcium phosphate from an amorphous calcium phosphate formulation in contact with a tooth in an oral cavity, the device comprising a first electrode and a second electrode spatially separated from the first electrode, wherein both the first electrode and the second electrode are arranged to contact the amorphous calcium phosphate formulation when the first electrode is arranged proximal to the tooth during use of the device; and the second electrode comprises a sacrificial material at least on its surface.

The present invention is based on the insight that an ACP formulation may be bought into contact with an electrode pair to trigger an electrochemical reaction causing the formation of hydroxide anions (OH⁻ ions) at the first electrode, e.g. the cathode, to locally increase the pH in the vicinity of the tooth and promote calcium phosphate deposition as disclosed by Zhang et al. However, rather than using electrophoresis in which the second electrode is located external to the oral cavity, the second electrode, e.g. the anode, is also brought into contact with the ACP formulation, preferably such that the tooth is located outside the current path between the first electrode and the second electrode, that is, the first electrode preferably is located between the tooth and the second electrode. This has the advantage that an unobstructed and therefore highly conductive current path is provided between the first and the second electrode, allowing for higher currents to flow between the first and second electrode compared to arrangements in which a tooth or another part of the body is located in between the electrodes. This further promotes calcium phosphate deposition due to the further increase in pH at the first electrode, as the rate of formation of hydroxide anions is proportional to the electrical current flow. Hence, when the first electrode and the second electrode are conductively coupled to a power source such as a battery or the like, the potential difference across the first electrode and the second electrode will cause an electrochemical half-reaction at both the first electrode and the second electrode. As it is undesirable to have the generation of protons (H⁺ ions) in relative close vicinity to the first electrode, as this could neutralize the generated hydroxide ions at the first electrode, the second electrode comprises a sacrificial material on at least its surface, e.g. the second electrode comprises a sacrificial material coating or is made of a sacrificial material, such that the electrochemical half-reaction at the second electrode involves the formation of cations of the sacrificial material rather than the formation of protons, thereby preventing neutralizing the hydroxide ions formed at the first electrode such that calcium phosphate deposition on and in the tooth surface is promoted by the increased pH local to the first electrode, in particular where active or passive diffusion of calcium and phosphate ions into to the porous tooth structure is facilitated prior to application of the potential difference across the electrodes. This for example may be achieved by using an ACP formulation having a pH that is low enough to suppress spontaneous calcium phosphate precipitation from the formulation.

The sacrificial material may be any suitable material, such as magnesium, aluminum or zinc. Preferably, the sacrificial material is zinc or a zinc alloy as zinc has a particularly low oxidation potential and zinc ions have an antimicrobial effect, thus giving the additional advantage of killing bacteria residing in the carious structures.

The first electrode preferably contacts the tooth surface of the tooth to be remineralized. In order to achieve a particularly large contact surface between the first electrode and the tooth surface, the first electrode preferably a brush electrode. Such a brush electrode may be made of any suitable material, with a carbon material, e.g. graphite, being particularly suitable.

In a preferred set of embodiments, the oral treatment device further comprises a reservoir for containing the amorphous calcium phosphate formulation such that the ACP formulation may be applied to the tooth with the oral treatment device.

Preferably, the first electrode and the second electrode are contained within the reservoir. For example, the reservoir may be a dental tray comprising a plurality of the first electrodes and a plurality of the second electrodes arranged in pairs of electrodes each comprising one of the first electrodes and one of the second electrodes, each pair of electrodes being arranged to stimulate deposition of calcium phosphate from the amorphous calcium phosphate formulation on a different tooth within the oral cavity. This facilitates a particularly user-friendly and efficient application of the ACP formulation to the teeth in the oral cavity. The dental tray may be made of an elastomeric material, e.g. a rubber-like material, to allow the dental tray to mold its shape around the teeth of the patient or user, thereby ensuring a comfortable fit of the dental try within the oral cavity.

Alternatively, the oral treatment device is an applicator having a nozzle fluidly coupled to a coupling member arranged to interface with a reservoir, wherein the first electrode is arranged proximal to an orifice of the nozzle and the second electrode is arranged upstream from the first electrode on the nozzle, which has the advantage that specific teeth within the oral cavity may be targeted using such an applicator. In such an oral treatment device, the reservoir may form an integral part of the oral treatment device, e.g. may form a removable container or the like, or alternatively the reservoir may be a separate entity, e.g. a disposable cartridge or the like, that may be coupled to the nozzle through the coupling member.

In an alternative set of embodiments, the oral treatment device is an electric toothbrush or an oral irrigator, in which case the application of the ACP formulation within the oral cavity may be performed prior to contacting the ACP formulation with the electrode pair(s).

The oral treatment device further comprises a power source coupled to each first electrode and each second electrode such that during use of the oral treatment device each first electrode acts as a cathode and each second electrode acts as an anode, and the power source is arranged to apply a potential difference across each pair of anodes and cathodes causing a pH increase at each cathode through the formation of hydroxide anions; and the formation of cations of the sacrificial material at the anode as previously explained. Such a potential difference advantageously may be in a range of 0.5-2 V. If the potential difference is below 0.5 V, the respective reaction rates of the electrochemical half-reactions may be too low, whereas when the potential difference is above 2 V, undesirable competing half-reactions may start to occur, such as competing acid formation at the second electrode, as well as noticeable discomfort to users where the electrical currents generated by such potential differences penetrate the teeth or body of such users.

According to another aspect, there is provided a tooth remineralization kit comprising the oral treatment device of any of the herein described embodiments and an amorphous calcium phosphate formulation. Such a tooth mineralization kit can be advantageously used to effectively deposit calcium phosphate in and on tooth surfaces, e.g. to protect the tooth from the onset of caries.

The amorphous calcium phosphate formulation may be a single phase formulation preferably having a pH in a range of 4-6. This has the advantage that no mixing of different phases of the formulation is required, whereas a pH in this range ensures minimal precipitation of calcium phosphate from the formulation, such that this precipitation can be effectively triggered by the pH increase at the first electrode once the ACP formulation is applied to the tooth or teeth to be treated.

According to yet another aspect, there is provided an amorphous calcium phosphate formulation for use in a method for remineralizing a tooth using an amorphous calcium phosphate formulation, the method comprising applying the amorphous calcium phosphate formulation to the tooth; contacting the applied amorphous calcium phosphate formulation with a first electrode proximal to the tooth and a second electrode, the second electrode comprising a sacrificial material at least on its surface; and applying a potential difference across the first electrode and the second electrode causing an increase in pH at the first electrode though the formation of hydroxide anions at the first electrode and the formation of cations of the sacrificial material at the second electrode.

### BRIEF DESCRIPTION OF THE DRAWINGS

Embodiments of the invention are described in more detail and by way of non-limiting examples with reference to the accompanying drawings, wherein:
FIG. 1 is a scanning electron microscope image of dentin;
FIG. 2 is a scanning electron microscope image of carious enamel;
FIG. 3 schematically depicts a cross-sectional view of an oral treatment device according to an embodiment;
FIG. 4 schematically depicts a perspective view of an oral treatment device according to another embodiment;
FIG. 5 schematically depicts a cross-sectional view of an oral treatment device according to yet another embodiment;
FIG. 6 schematically depicts a cross-sectional view of an oral treatment device according to yet another embodiment;
FIG. 7 schematically depicts a cross-sectional view of an oral treatment device according to yet another embodiment;
FIG. 8 is a flowchart of a method in which an amorphous calcium phosphate formulation can be used; and
FIGS. 9-11 are images depicting experimental results demonstrating proof of concept of the present invention.

### DETAILED DESCRIPTION OF THE EMBODIMENTS

It should be understood that the Figures are merely schematic and are not drawn to scale. It should also be understood that the same reference numerals are used throughout the Figures to indicate the same or similar parts.

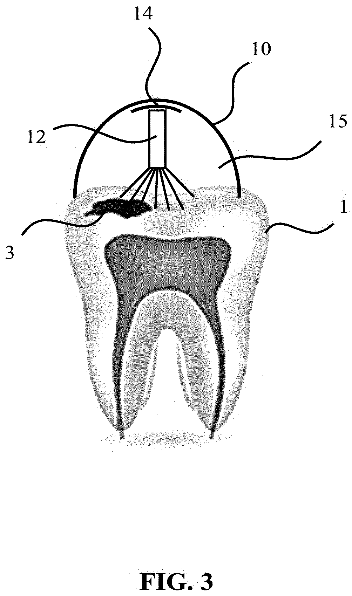

FIG. 3 schematically depicts an oral treatment device 10 according to an embodiment. In this embodiment, the oral treatment device 10 is an applicator that can be positioned over at least one tooth 1 to be treated with an ACP formulation, e.g. because the tooth 1 exhibits the onset of caries defect 3. Of course, the tooth 1 may be treated for a number of reasons, e.g. to counter tooth sensitivity, such as tooth sensitivity resulting from a tooth whitening treatment or from enamel erosion through prolonged exposure to acid environments. The applicator typically contains a first electrode 12 arranged to be located in close proximity to the tooth 1 to be treated. The first electrode 12 preferably contacts the tooth 1. In order to achieve a large contact area between the first electrode 12 and the tooth 1, the first electrode 12 advantageously may take the shape of a brush electrode, which may be made of any suitable material. A particularly suitable material is a carbon-based material such as graphite.

The applicator further comprises a second electrode 14 that is spaced apart from the first electrode 12 such that a short-circuit between the first electrode and the second electrode 14 is avoided. The second electrode 14 may be located in any suitable location within the applicator. For example, the second electrode 14 may be positioned such that the tooth 1 is located in between the first electrode 12 and the second electrode 14, or may positioned such that the tooth 1 is outside a direct path between the first electrode 12 and the second electrode 14, e.g. the first electrode 12 is positioned in between the second electrode 14 and the tooth 1. More generally, the second electrode 14 may be located further away from the tooth 1 than the first electrode 12. For reasons explained in further detail below, the second electrode 14 contains a sacrificial material at least on its surface, that is, the second electrode 14 is coated with a sacrificial material or is formed of such a sacrificial material. Any suitable sacrificial material may be used for this purpose, and non-limiting examples of suitable sacrificial materials include magnesium, aluminum and zinc. Zinc (and zinc alloys) are particularly suitable because zinc cations are known to have an antimicrobial effect, such that the generation of such zinc cations in the vicinity of the tooth 1 can assist in killing harmful bacteria present on or in the tooth 1, e.g. in the caries defect 3.

The first electrode 12 and second electrode 14 are located in a reservoir 15 of the applicator for containing the ACP formulation such that both the first electrode 12 and the second electrode 14 contact the ACP formulation when loaded into the reservoir 15 of the applicator. During use of the applicator, the first electrode 12 and the second electrode 14 are conductively coupled to a power source (not shown) such as a battery or the like such that the first electrode 12 acts as the negative electrode (cathode) and the second electrode 14 acts as the positive electrode (anode). The power source may form an integral part of the applicator or may be attached to the applicator prior to use, e.g. through leads (not shown) extending from the applicator, which each lead being conductively coupled to one of the first electrode 12 and the second electrode 14. The power source creates a potential difference across the first electrode 12 and the second electrode 14 that triggers the following electrochemical half-reactions in case of the second electrode 14 comprising Zinc as its sacrificial material:

| | |
|---|---|
| 2H₂O(l) + 2e⁻ 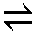 H₂(g) + 2OH⁻ | -0.8277 V (Cathode) |
| Zn²⁺ (aq) + 2e⁻ 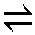 Zn(s) | -0.7618 V (Anode) |

Of course, the anode half-reaction may involve other sacrificial materials instead as will be readily understood by the skilled person. Generally speaking, any biocompatible sacrificial material that has a standard electrode potential lower than that of water for its redox reaction may be used for the second electrode 14 in the oral treatment device 10. The water hydrolyzed at the first electrode may come from any suitable source, e.g. water contained in the ACP formulation.

Importantly, the use of a sacrificial material for the second electrode 14 causes this material to become a reagent in the electrochemical half-reaction at the second electrode 14, thereby promoting the formation of cations of the sacrificial material and suppressing the formation of protons (H⁺ ions) from water hydrolysis. This therefore suppresses the unwanted recombination of hydroxide anions and protons into water molecules, which would counter the pH increase at the first electrode 12 by the generated hydroxide anions, which pH increase is required to increase the precipitation rate of calcium phosphate in or on the tooth surface. Moreover, the generation of protons is generally undesirable as acidic conditions should be avoided within an oral cavity, as such acidic conditions can cause enamel erosion as is well-known per se.

Another advantage of the use of a sacrificial material for the second electrode 14 is that the electrochemical reaction will commence at very low voltages, e.g. 0.1V, although voltages of above 0.5V are preferred to ensure sufficient deposition rates of the calcium phosphate. In an example embodiment, the power source is arranged to create a potential difference across the first electrode 12 and the second electrode 14 in a range of 1 - 2 V, thus ensuring a long continuous operation time in case of the power source comprising one or more batteries as well as minimizing the risk of the user of the oral treatment device 10 experiencing any discomfort.

Preferably, the potential difference is applied across the first electrode 12 and the sacrificial second electrode 14 of the oral treatment device 10 after a short delay following application of the ACP formulation onto the tooth 1 to allow diffusion of calcium, phosphate and, if present in the formulation, fluoride ions into the porous structures of the tooth 1. Such ion transport may be effected by passive diffusion, e.g. over a period of a few minutes, or by active delivery. For example, where the first electrode 12 and the second electrode 14 are arranged in a suitable configuration, iontophoresis may be applied using these electrodes to invoke such ion transport prior to the commencement of the aforementioned electrochemical reaction to stimulate calcium phosphate precipitation. Other suitable active ion transport techniques will be apparent to the skilled person.

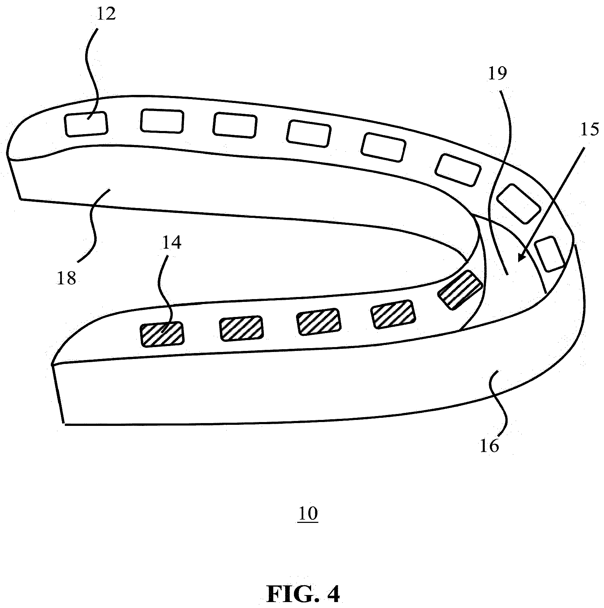

The applicator may be made of any suitable material. Preferably, the applicator is made of an elastomeric material, e.g. a rubber-like material such as a silicone-based elastomer such that a malleable applicator is provided that can be easily molded to fit the shape of the tooth 1. Depending on the viscosity of the ACP formulation, the applicator may not be fully closed, as this for instance is unnecessary when the ACP formulation is highly viscous. In an embodiment, the oral treatment device 10 in the form of an applicator is a dental tray as schematically depicted in FIG. 4. The dental tray may comprise a plurality of first electrodes 12, e.g. arranged on a first wall section 16 of the dental tray and a plurality of sacrificial second electrodes 14, e.g. arranged on a second wall section 18 of the dental tray opposing the first wall section 16. Of course, other electrode arrangements, e.g. where the second electrodes 14 are arranged on a bottom section 19 of the dental tray, which bottom section 19 connects the first wall section 16 to the second wall section 18. The reservoir 15 is typically enclosed by the first wall section 16, the opposing second wall section 18 and the bottom section 19 of the dental tray. It should be understood that these sections may not be discrete portions of the dental tray, i.e. the dental tray may be a molded device, e.g. molded from an elastomeric material as previously explained.

The plurality of first electrodes 12 and the plurality of sacrificial second electrodes 14 may be arranged in electrode pairs with each pair consisting of a first electrode 12 and a second electrode 14 arranged to treat one of the teeth to be fitted into the dental tray such that each tooth 1 is aligned with one of these electrode pairs. The power source (not shown) may be arranged to address each of these pairs simultaneously or alternatively may be configurable to address individual electrode pairs, e.g. where specific teeth are targeted. To this end, a control device (not shown) for controlling the power source may visualize the teeth to be fitted into the dental tray such that a user of the control device can select the teeth to be treated, which user selection is translated by the control device into a control instruction to select the electrode pairs corresponding to the selected teeth such that ACP precipitation is stimulated at the selected teeth only by selective application of the potential difference across the electrode pairs associated with the selected teeth.

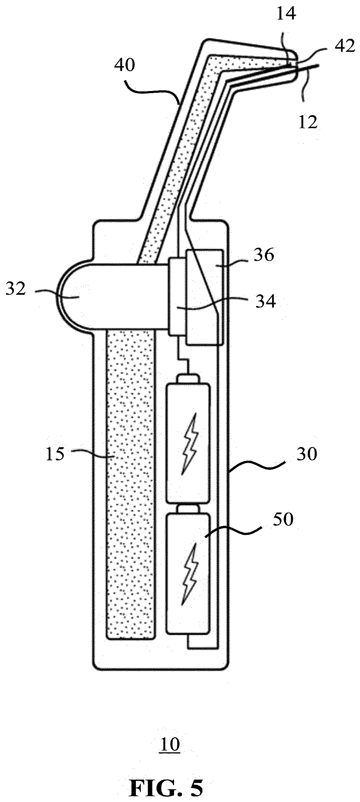

FIG. 5 schematically depicts another example embodiment of an oral treatment device 10 in the form of an applicator. In this embodiment, the applicator is a handheld device comprising a body 30 containing the reservoir 15, which reservoir 15 is fluidly connected to a nozzle 40 having an orifice 42 through which the ACP formulation within the reservoir 15 can be applied to the patient's teeth. The ACP formulation may be forced from the reservoir 15 through the nozzle orifice 42 by a pump 32, e.g. a manual pump that can be activated by digit pressure such as thumb pressure. The pump 32 may activate a switch 34, which in turn activates a controller 36 controlling the delivery of a potential difference from the power source 50 across the first electrode 12 and the sacrificial second electrode 14 that are both arranged proximal to the nozzle orifice 42. The first electrode 12 typically extends further away from the nozzle 40 than the second electrode 14, i.e. the second electrode 14 is arranged upstream to the first electrode 12, such that the first electrode 12 may contact the surface of a tooth to be treated whilst the second electrode, whilst still in contact with the deposited ACP formulation is further away from the tooth to be treated than the first electrode 12.

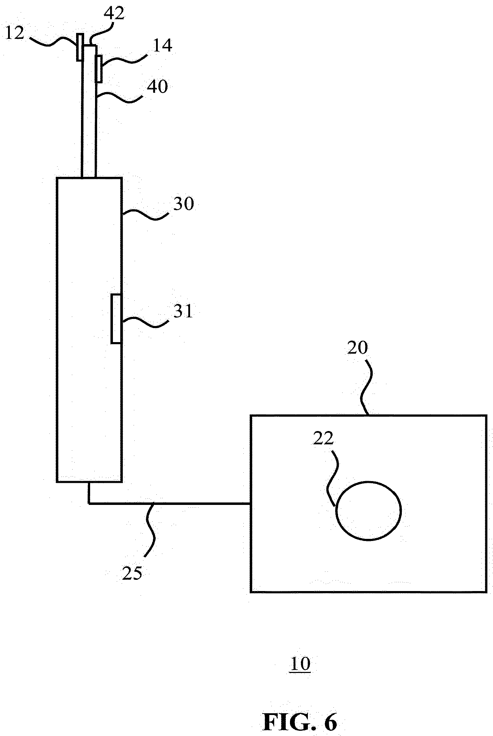

Although the oral treatment device 10 in preferred embodiments is an applicator, more preferably a dental dray, other embodiments are also feasible. For example, the oral treatment device 10 may be an oral irrigator as schematically depicted in FIG. 6, e.g. an air floss device or a water jet device. Such an oral irrigator may comprise a base unit 20 and a handheld unit 30 fluidly connected to the base unit 20 through tubing 25. The base unit 20 may comprise a user interface 22 such as a dial or knob through which a user may select a mode of operation of the oral treatment device 10. The handheld unit 30 comprises a nozzle arrangement 40 through which a fluid stream may be aimed at an interdental region such as a subgingival region to be cleaned by the user. The handheld unit 30 may comprise a switch 32 for enabling/disabling such a fluid stream. In alternative embodiments, the base unit 20 may be omitted as a separate entity and its functionality may be integrated in the handheld unit 30. The first electrode 12 and the sacrificial second electrode 14 may be arranged proximal to the nozzle arrangement orifice 42, with the first electrode 12 typically extending further away from the nozzle arrangement than the second electrode 14 such that the first electrode 12 can contact an ACP formulation separately applied to the teeth of a user proximal to the user's tooth whilst the second electrode 14 is also arranged to contact the ACP formulation but at a greater distance from the user's tooth than the first electrode 14. The oral irrigator may have a dedicated mode of operation in which the fluid stream is disabled but the provision of the potential difference across the first electrode 12 and the second electrode 14 is enabled such as to stimulate precipitation of calcium phosphate from the ACP formulation as previously explained without disturbing the applied ACP formulation by generation of a fluid stream.

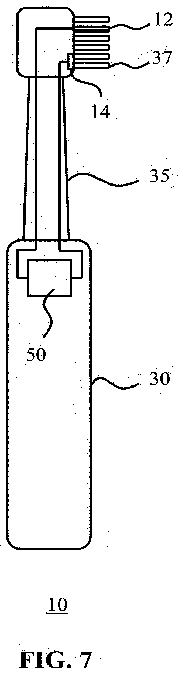

The oral treatment device 10 may also take the form of an electric toothbrush, e.g. a sonic toothbrush, as schematically depicted in FIG. 7. In this embodiment, the first electrode 12 and the sacrificial second electrode 14 may be positioned in the (detachable) brush head 35 of the electric toothbrush, with the (rechargeable) power source 50 being located in the base of the electric toothbrush. For example, the first electrode 12 may be located in amongst the brush hairs 37 of the brush head 35, whereas the second electrode 14 may be located at the interface between the brush hairs 37 and the brush head 35 such that the first electrode 12 can contact an ACP formulation separately applied to the teeth of a user proximal to the user's tooth whilst the second electrode 14 is also arranged to contact the ACP formulation but at a greater distance from the user's tooth than the first electrode 14. The electric toothbrush may have a dedicated mode of operation in which the brush head is disabled but the provision of the potential difference across the first electrode 12 and the second electrode 14 is enabled such as to stimulate precipitation of calcium phosphate from the ACP formulation as previously explained without disturbing the applied ACP formulation by brushing movements.

An ACP formulation activation kit may be provided by combining the oral treatment device 10 according to any of the herein described embodiments with an ACP formulation, e.g. a CPP-ACP formulation or any other suitable ACP formulation. The ACP formulation preferably is a single-phase composition comprising both the calcium source, e.g. CaCl₂, and the phosphate source, e.g. NaH₂PO₄. For example, an equimolar mixture of CaCl₂ and NaH₂PO₄ yields a formulation having a pH of around 4.2 whereas NaH₂PO₄ can be mixed Na₂HPO₄ with to increase the pH of the formulation, which may be desirable as a formulation that is too acidic can cause enamel erosion. It was found that by mixing NaH₂PO₄ with Na₂HPO₄ the pH of the formulation could be increased to 5.5 without significant calcium phosphate precipitation occurring in the formulation. The pH of the single phase formulation may be in a range of 4-6 and preferably is in a range of 4.5 - 5.5 to prevent significant calcium phosphate precipitation in the formulation whilst preventing the formulation from becoming overly acidic, which may be detrimental to the teeth to which the formulation is applied, especially when the formulation is left in situ for a period of time prior to activation of the electrochemical reaction with the oral treatment device 10 causing the pH local to the teeth to increase to around 8.5 by the formation of hydroxide ions at the first electrode 12 as previously explained. Of course, other phosphate salts, e.g. potassium salts, and other calcium salts, e.g. nitrate salts may be used in the ACP formulation. In addition, fluorides, thickeners, scaffold formers such as peptides, preservatives, taste components and so on may be added to the ACP formulation. In a particularly advantageous embodiment, the ACP formulation comprises KNO₃, which increases the electrical conductivity of the ACP formulation and reduces nerve sensitivity of (sensitive) teeth.

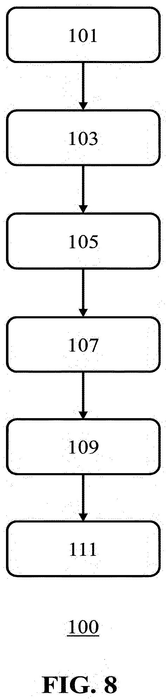

FIG. 8 is a flowchart of a method 100 in which an amorphous calcium phosphate formulation can be used. The method 100 starts in operation 101 with the provision of an ACP formulation and an oral treatment device 10 according to any of the herein described embodiments, e.g. in the form of a kit of parts, or separately. In operation 103, the ACP formulation is applied to the one or more teeth to be remineralized, e.g. with the oral treatment device 10 in case the oral treatment device 10 is an applicator such as a dental tray, or separately. The method 100 preferably further comprises optional operation 105, in which the calcium, phosphate and, if present, fluoride ions of the applied ACP formulation are allowed to transport into the porous tooth structure, e.g. by diffusion or by means of active ion transport such as through iontophoresis.

Next, in operation 107, the applied amorphous calcium phosphate formulation is contacted by the first electrode 12 proximal to the tooth to be treated and by the sacrificial second electrode 14 of the oral treatment device 10, after which the method 100 proceeds to operation 109 in which a potential difference is applied across the first electrode 12 and the sacrificial second electrode 14 of the oral treatment device 10 causing an increase in pH at the first electrode 12 though the formation of hydroxide anions and the formation of cations of the sacrificial material at the second electrode 14. The local increase of the pH at the first electrode 12 in close proximity to the tooth to be treated stimulates the precipitation of calcium phosphate in the pores and on the surface of the tooth, thereby remineralizing the tooth. Upon termination of the applied potential difference, the method 100 terminates in operation 111.

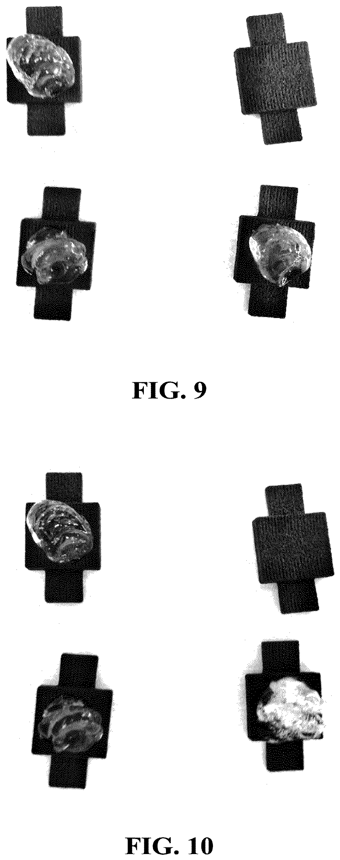

In order to demonstrate proof of concept, an experiment was conducted in which a commercially available ACP formulation was deposited on three sintered porous polyamide-12 tiles mimicking a porous tooth surface as shown in FIG. 9. A fourth sintered porous polyamide-12 tile (top right corner) was left uncovered as a negative control. In the experiment, the ACP formulation on the tile in the top left corner was used as a positive control, the ACP formulation on the tile in the bottom left corner was used as per the directions provided with the commercially available ACP formulation and the ACP formulation in the bottom left corner was contacted with a Pt electrode (cathode) and a Zn electrode (anode) across which a voltage of 1.5V was applied corresponding to a current of approximately 6mA, causing immediate bubbling at the Pt electrode and white precipitation on the tile. The results after 30 minutes are shown in FIG. 10, which clearly shows a significant increase in calcium phosphate precipitation on the bottom right tile exposed to the electrochemical reaction compared to the bottom left tile for which the directions of use of the ACP formulation were followed and the top left tile (positive control tile).

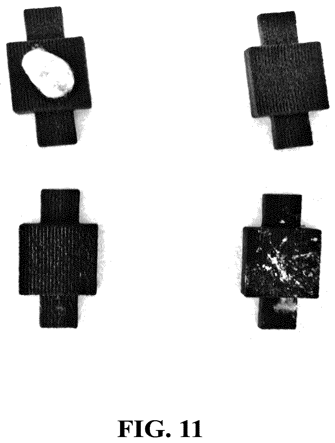

After 30 minutes, the gels were rinsed off the bottom left and bottom right tiles using slowly running tap water to mimic a user drinking, and wiped with a tissue to mimic the action of the user's tongue. The results are shown in FIG. 11. Whilst the bottom left tile showed no visible deposits, the bottom right tile clearly visible deposits can be observed, thereby demonstrating that the exposure of the ACP formulation to the electrochemical reaction significantly enhances calcium phosphate deposition compared to the directed use of the ACP formulation. The positive control tile (top left corner) was simply dried over 1 week, thereby showing the maximum possible deposition from the ACP formulation.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. Measures are recited in mutually different dependent claims can advantageously be combined.

## Claims

1. An oral treatment device (10) for stimulating deposition of calcium phosphate from an amorphous calcium phosphate formulation in contact with a tooth (1) in an oral cavity, the device comprising a first electrode (12), a second electrode (14) spatially separated from the first electrode, and a power source (50) coupled to the first electrode and the second electrode such that, during use of the device, the first electrode acts as a cathode and the second electrode acts as an anode, wherein:
both the first electrode and the second electrode are arranged to contact the amorphous calcium phosphate formulation when the first electrode is arranged proximal to the tooth during use of the device;
the second electrode comprises a sacrificial material at least on its surface, and **characterized in that** the power source is arranged to apply a potential difference across the anode and the cathode, which potential difference is sufficient to cause:
a pH increase at the cathode through the formation of hydroxide anions; and
the formation of cations of the sacrificial material at the anode.

2. The oral treatment device (10) of claim 1, wherein the sacrificial material is zinc or a zinc alloy.

3. The oral treatment device (10) of claim 1 or 2, wherein the first electrode (12) is a brush electrode.

4. The oral treatment device (10) of claim 3, wherein the brush electrode is made of carbon.

5. The oral treatment device (10) of any of claims 1-4, further comprising a reservoir (15) for containing the amorphous calcium phosphate formulation.

6. The oral treatment device (10) of claim 5, wherein the first electrode (12) and the second electrode (14) are contained within the reservoir (15).

7. The oral treatment device (10) of claim 5 or 6, wherein the reservoir is a dental tray comprising a plurality of the first electrodes (12) and a plurality of the second electrodes (14) arranged in pairs of electrodes each comprising one of the first electrodes and one of the second electrodes, each pair of electrodes being arranged to stimulate deposition of calcium phosphate from the amorphous calcium phosphate formulation on a different tooth (1) within the oral cavity.

8. The oral treatment device (10) of claim 7, wherein the dental tray is made of an elastomeric material.

9. The oral treatment device (10) of any of claims 1-4, wherein the oral treatment device is an applicator having a nozzle (40) fluidly coupled to a coupling member arranged to interface with a reservoir (15), wherein the first electrode (12) is arranged proximal to an orifice (42) of the nozzle and the second electrode (14) is arranged upstream from the first electrode on the nozzle.

10. The oral treatment device (10) of any of claims 1-4, wherein the oral treatment device is an electric toothbrush or an oral irrigator.

11. The oral treatment device (10) of any of claims 1-10, wherein the potential difference is in a range of 0.5-2 V.

12. A tooth remineralization kit comprising the oral treatment device (10) of any of claims 1-11 and an amorphous calcium phosphate formulation.

13. An amorphous calcium phosphate formulation for use in a remineralizing treatment of a tooth comprising applying (103) the amorphous calcium phosphate formulation to the tooth (1); contacting (107) the applied amorphous calcium phosphate formulation with a first electrode (12) proximal to the tooth and a second electrode (14), the second electrode comprising a sacrificial material at least on its surface; and **characterized by** applying (109) a potential difference across the first electrode and the second electrode causing an increase in pH at the first electrode though the formation of hydroxide anions and the formation of cations of the sacrificial material at the second electrode.

14. The tooth remineralization kit of claim 12, or the amorphous calcium phosphate formulation as claimed in claim 13, wherein the amorphous calcium phosphate formulation is a single-phase formulation preferably having a pH in a range of 4-6.

## Patentansprüche

1. Orale Behandlungsvorrichtung (10) zum Stimulieren der Ablagerung von Kalziumphosphat aus einer amorphen Kalziumphosphatformulierung in Kontakt mit einem Zahn (1) in einer oralen Kavität, wobei die Vorrichtung eine erste Elektrode (12), eine zweite Elektrode (14), die räumlich von der ersten Elektrode getrennt ist, und eine Leistungsquelle (50) umfasst, die mit der ersten Elektrode und der zweiten Elektrode gekoppelt ist, so dass während der Verwendung der Vorrichtung die erste Elektrode als eine Kathode und die zweite Elektrode als eine Anode wirkt, wobei:
sowohl die erste Elektrode als auch die zweite Elektrode angeordnet sind, um die amorphe Kalziumphosphatformulierung zu kontaktieren, wenn die erste Elektrode während der Verwendung der Vorrichtung proximal zum Zahn angeordnet ist;
die zweite Elektrode mindestens auf ihrer Oberfläche ein Opfermaterial umfasst, und **dadurch gekennzeichnet, dass** die Leistungsquelle angeordnet ist, um eine Potentialdifferenz zwischen der Anode und der Kathode anzulegen, wobei die Potentialdifferenz ausreichend ist, um Folgendes zu verursachen:
eine pH-Wert-Erhöhung an der Kathode durch die Bildung von Hydroxidanionen; und
die Bildung von Kationen des Opfermaterials an der Anode.

2. Orale Behandlungsvorrichtung (10) nach Anspruch 1, wobei das Opfermaterial Zink oder eine Zinklegierung ist.

3. Orale Behandlungsvorrichtung (10) nach Anspruch 1 oder 2, wobei die erste Elektrode (12) eine Bürstenelektrode ist.

4. Orale Behandlungsvorrichtung (10) nach Anspruch 3, wobei die Bürstenelektrode aus Kohlenstoff gemacht ist.

5. Orale Behandlungsvorrichtung (10) nach einem der Ansprüche 1-4, weiter umfassend ein Reservoir (15) zum Enthalten der amorphen Kalziumphosphatformulierung.

6. Orale Behandlungsvorrichtung (10) nach Anspruch 5, wobei die erste Elektrode (12) und die zweite Elektrode (14) innerhalb des Reservoirs (15) enthalten sind.

7. Orale Behandlungsvorrichtung (10) nach Anspruch 5 oder 6, wobei das Reservoir eine Dentalschiene ist, die eine Vielzahl von ersten Elektroden (12) und eine Vielzahl von zweiten Elektroden (14) umfasst, die in Paaren von Elektroden angeordnet sind, von denen jedes eine der ersten Elektroden und eine der zweiten Elektroden umfasst, wobei jedes Paar von Elektroden angeordnet ist, um die Ablagerung von Kalziumphosphat aus der amorphen Kalziumphosphatformulierung an einem anderen Zahn (1) innerhalb der oralen Kavität zu stimulieren.

8. Orale Behandlungsvorrichtung (10) nach Anspruch 7, wobei die Dentalschiene aus einem Elastomermaterial gemacht ist.

9. Orale Behandlungsvorrichtung (10) nach einem der Ansprüche 1-4, wobei die orale Behandlungsvorrichtung ein Applikator ist, der eine Düse (40) aufweist, die fluidisch mit einem Kopplungsglied gekoppelt ist, das angeordnet ist, um mit einem Reservoir (15) in Verbindung zu stehen, wobei die erste Elektrode (12) proximal zu einer Öffnung (42) der Düse angeordnet ist und die zweite Elektrode (14) stromaufwärts von der ersten Elektrode an der Düse angeordnet ist.

10. Orale Behandlungsvorrichtung (10) nach einem der Ansprüche 1-4, wobei die orale Behandlungsvorrichtung eine elektrische Zahnbürste oder eine Munddusche ist.

11. Orale Behandlungsvorrichtung (10) nach einem der Ansprüche 1-10, wobei die Potentialdifferenz in einem Bereich von 0,5-2 V ist.

12. Zahnremineralisierungskit, umfassend die orale Behandlungsvorrichtung (10) nach einem der Ansprüche 1-11 und eine amorphe Kalziumphosphatformulierung.

13. Amorphe Kalziumphosphatformulierung zur Verwendung in einer Remineralisierungsbehandlung eines Zahns, umfassend Aufbringen (103) der amorphen Kalziumphosphatformulierung auf den Zahn (1); Kontaktieren (107) der aufgebrachten amorphen Kalziumphosphatformulierung mit einer ersten Elektrode (12) proximal zum Zahn und einer zweiten Elektrode (14), wobei die zweite Elektrode mindestens auf ihrer Oberfläche ein Opfermaterial umfasst; und **gekennzeichnet durch** Anlegen (109) einer Potentialdifferenz zwischen der ersten Elektrode und der zweiten Elektrode, was eine Erhöhung des pH-Wertes an der ersten Elektrode durch die Bildung von Hydroxidanionen und die Bildung von Kationen des Opfermaterials an der zweiten Elektrode verursacht.

14. Zahnremineralisierungskit nach Anspruch 12 oder die amorphe Kalziumphosphatformulierung nach Anspruch 13, wobei die amorphe Kalziumphosphatformulierung eine einphasige Formulierung ist, die vorzugsweise einen pH-Wert im Bereich von 4-6 aufweist.

## Revendications

1. Dispositif de traitement buccal (10) pour stimuler le dépôt de phosphate de calcium à partir d'une formulation de phosphate de calcium amorphe en contact avec une dent (1) dans une cavité buccale, le dispositif comprenant une première électrode (12), une seconde électrode (14) spatialement séparée de la première électrode, et une source d'énergie (50) couplée à la première électrode et à la seconde électrode de telle sorte que, pendant l'utilisation du dispositif, la première électrode agisse comme une cathode et la seconde électrode agisse comme une anode, dans lequel :
la première électrode et la seconde électrode sont toutes deux agencées pour entrer en contact avec la formulation de phosphate de calcium amorphe lorsque la première électrode est agencée à proximité de la dent pendant l'utilisation du dispositif ;
la seconde électrode comprend un matériau sacrificiel au moins sur sa surface, et **caractérisé en ce que** la source d'énergie est agencée pour appliquer une différence de potentiel entre l'anode et la cathode, laquelle différence de potentiel est suffisante pour provoquer :
une augmentation de pH à la cathode par la formation d'anions hydroxyde ; et
la formation de cations du matériau sacrificiel à l'anode.

2. Dispositif de traitement buccal (10) selon la revendication 1, dans lequel le matériau sacrificiel est du zinc ou un alliage de zinc.

3. Dispositif de traitement buccal (10) selon la revendication 1 ou 2, dans lequel la première électrode (12) est une électrode-balai.

4. Dispositif de traitement buccal (10) selon la revendication 3, dans lequel l'électrode-balai est en carbone.

5. Dispositif de traitement buccal (10) selon l'une quelconque des revendications 1-4, comprenant en outre un réservoir (15) destiné à contenir la formulation de phosphate de calcium amorphe.

6. Dispositif de traitement buccal (10) selon la revendication 5, dans lequel la première électrode (12) et la seconde électrode (14) sont contenues dans le réservoir (15).

7. Dispositif de traitement buccal (10) selon la revendication 5 ou 6, dans lequel le réservoir est un plateau dentaire comprenant une pluralité de premières électrodes (12) et une pluralité de secondes électrodes (14) agencées par paires d'électrodes comprenant chacune l'une des premières électrodes et l'une des secondes électrodes, chaque paire d'électrodes étant agencée pour stimuler le dépôt de phosphate de calcium à partir de la formulation de phosphate de calcium amorphe sur une dent différente (1) dans la cavité buccale.

8. Dispositif de traitement buccal (10) selon la revendication 7, dans lequel le plateau dentaire est constitué d'une matière élastomère.

9. Dispositif de traitement buccal (10) selon l'une quelconque des revendications 1-4, dans lequel le dispositif de traitement buccal est un applicateur présentant une buse (40) couplée fluidiquement à un élément de couplage agencé pour s'interfacer avec un réservoir (15), dans lequel la première électrode (12) est agencée à proximité d'un orifice (42) de la buse et la seconde électrode (14) est agencée en amont de la première électrode sur la buse.

10. Dispositif de traitement buccal (10) selon l'une quelconque des revendications 1-4, dans lequel le dispositif de traitement buccal est une brosse à dents électrique ou un irrigateur buccal.

11. Dispositif de traitement buccal (10) selon l'une quelconque des revendications 1-10, dans lequel la différence de potentiel est comprise dans une plage de 0,5-2 V.

12. Kit de reminéralisation des dents comprenant le dispositif de traitement buccal (10) selon l'une quelconque des revendications 1-11 et une formulation de phosphate de calcium amorphe.

13. Formulation de phosphate de calcium amorphe destinée à être utilisée dans un traitement de reminéralisation d'une dent, comprenant l'application (103) de la formulation de phosphate de calcium amorphe sur la dent (1) ; la mise en contact (107) de la formulation de phosphate de calcium amorphe appliquée avec une première électrode (12) à proximité de la dent et une seconde électrode (14), la seconde électrode comprenant un matériau sacrificiel au moins sur sa surface ; et **caractérisée par** l'application (109) d'une différence de potentiel entre la première électrode et la seconde électrode provoquant une augmentation du pH à la première électrode par la formation d'anions hydroxyde et la formation de cations du matériau sacrificiel à la seconde électrode.

14. Kit de reminéralisation des dents selon la revendication 12, ou formulation de phosphate de calcium amorphe selon la revendication 13, dans lequel ou laquelle la formulation de phosphate de calcium amorphe est une formulation monophasique présentant de préférence un pH dans une plage de 4-6.
